Europäisches Patentamt

European Patent Office

Office européen des brevets

(11) Publication number: **0 384 755**
**A1**

## (12) EUROPEAN PATENT APPLICATION

(21) Application number: 90301906.5

(22) Date of filing: 22.02.90

(51) Int. Cl.⁵: **B01J 27/28, B01J 38/06, B01J 38/12, C07C 51/377, C07C 57/04**

(30) Priority: 22.02.89 JP 40183/89

(43) Date of publication of application:
29.08.90 Bulletin 90/35

(84) Designated Contracting States:
DE FR

(71) Applicant: **NIPPON SHOKUBAI KAGAKU KOGYO CO. LTD.**
**1-1, Koraibashi, 4-chome**
**Chuo-ku Osaka-shi Osaka-fu(JP)**

(72) Inventor: **Hashiba, Hideto**
**931-11, Hamada, Aboshi-ku**
**Himeji-shi, Hyogo-ken(JP)**
Inventor: **Aoki, Yukio**
**336-8, Tobo, Taishi-cho**
**Ibo-gun, Hyogo-ken(JP)**

(74) Representative: **Myerscough, Philip Boyd et al**
**J.A.Kemp & Co. 14, South Square Gray's Inn**
**London, WC1R 5EU(GB)**

(54) Method for regenerating catalyst for oxidative dehydrogenation of isobutyric acid.

(57) A method for regenerating a catalyst based on a heteropoly acid containing phosphorus, molybdenum and vanadium and/or its alkali metal salt and/or their partial substitution type compounds which is used in the production of methacrylic acid or its lower esters by the oxidative-dehydrogenation of isobutyric acid or its lower esters, including a step of treating an activity-lowered catalyst with a gas containing 1 to 30 volume % of steam and 5 to 25 volume % of oxygen at a temperature falling in the range of 200 to 400°C.

EP 0 384 755 A1

# METHOD FOR REGENERATING CATALYST FOR OXIDATIVE DEHYDROGENATION OF ISOBUTYRIC ACID

The present invention relates to regeneration of heteropoly acid type catalysts for synthesis of methacrylic acid or its lower esters by the oxidative dehydrogenation of isobutyric acid or its lower esters.

More particularly, the present invention relates to a method for regenerating heteropoly acid type catalysts containing, as indispensable components, phosphorus, molybdenum and vanadium which were used in the oxidative dehydrogenation reaction of isobutyric acid or its lower esters and deactivation by treating with a steam and oxygen-cotaining gas at specified temperature conditions.

It is a generally-known conventional technique to catalytically oxydehydrogenate isobutyric acid or its lower esters into methacrylic acid or its lower esters in the presence of a catalyst based on a hetropoly acid containing, as indispensable components, phosphorus, molybdenum and vanadium and/or alkali metal salt and/or their partial substitution type compounds.

These heteropoly acid type catalysts are slowly deactivated with a reaction time and hence, in effect, it is attempted to maintain their activity by slowly increasing the reaction time. However, heteropoly acid type catalysts, generally speaking, are unstable at high temperature and when the reaction temperature is raised, the decomposition of heteropoly acids is caused with it, and it, in turn, causes a rapid deterioration of activity.

In such situation, if an effective regeneration method of heteropoly acid type catalysts is to be provided, it is not necessary to raise the reaction temperature on purpose, and it is considered very favorable in terms of extending the lifetime of catalyst and maintenance of long-term stabilized reaction.

Heretofore, as regeneration methods of heteropoly acid type catalysts used for production of methacrylic acid or its lower esters by the oxidative dehydrogenation reaction of isobutyric acid or tis lower esters there is known a method of subjecting the catlyst to oxidation treatment with an oxygen-containing gas as disclosed in EP Publication No. 255640. It is mentioned there that in this prior art it is necessary to expose the catalyst to oxidation condition for the catalyst regeneration by keeping the catalyst away from reduction conditions at all time in order to prevent the catalyst from deactivation and that consequently, oxidation treatment with the oxygen-containing gas, even in a short period of time, is effective as the regeneration method.

With the purpose of studying in detail the method disclosed in the prior art the instant inventors tried to conduct experiments by changing reaction conditions in various ways, but its regeneration effect was no great and the results obtained were all far from satisfactory.

According to the finding of the instant inventors the regeneration by oxidation treatment is most effective when the treating temperature is held considerably higher than the usual reaction temperature, and effect obtained cannot be said sufficient at milder temperature conditions close to the reaction temperature. In the case, however, of actual plants, even in a short time, it is troublesome and in addition, loses a time to raise and lower the reaction temperature for each regeneration treatment, and it is not practical. Under such circumstances, there was expected the advent of a method being capable of regeneration treatment at the same or much the same temperature as the reaction temperature.

On the basis of the aforesaid finding the instant inventors further studied a regeneration method of heteropoly acid type catalysts, in consequence of which surprisingly, they found the fact that when steam is made to coexist in the oxygen-containing gas, its regeneration effect goes marked.

Thus, according to the present invention there is provided a method for regenerating a catalyst based on a heteropoly acid containing, as indispensable components, phosphorus, molybdenum and vanadium and/or its alkali metal salt and/or their partial substitution type compounds which is used in the production of methacrylic acid or its lower esters by the oxidative-dehydrogenation of isobutyric acid or its lower esters, including a step of treating an activity-lowered catalyst with a gas containing 1 to 30 volume % of steam and 5 to 25 volume % of oxygen at a temperature falling in the range of 200 to 400 $^{\circ}$ C.

Superior advantages with the present invention, as specifically disclosed in the hereinbelow-described Examples, will be illustrated as following 4 points.

(i) It is not necessary to take out of a reactor the deactivated catalyst and hence, losses by wear and crack caused by putting in and out the catalyst can be eliminated.

(ii) Sufficient regeneration effect is observed by treatment effected at a temperature falling in the range of usual reaction temperatures. Consequently, in the case of regeneration treatment it is sufficient to apply the reaction temperature as such, and it is not necessary to consider the raising and lowering of the gas temperature.

(iii) Activity of regenerated catalyst holds long.

(iv) High regeneration effect is produced even if the treating time is a very short time.

The catalyst regeneration of the present inven tion is carried out by passing into a reactor a mixed gas of 1 to 30 volume %, preferably 3 to 20 volume %, of steam, 5 to 25 volume % of oxygen and other gases (inert gases, such as nitrogen, carbon dioxide gas and the like) at a temperature of 200 to 400° C, preferably 230 to 360° C under a normal pressure to 10 atms at a space velocity of 300 to 5,000 hr$^{-1}$ (STP). The regeneration treatment time is optionally determined according to the treatment temperature and treating gas composition, but usually several minutes to 50 hours, preferably scores of minutes to 30 hours, are employed.

Heteropoly acid type catalysts used in the present invention should favorably have a composition represented by the general formula

$$P_a \, Mo_b \, V_c \, X_d \, Y_e \, O_f$$

in which X is at least one element selected from alkali metal, thallium and alkaline earth metal elements, Y is at least one element selected from Cu, Ag, As, Sb, Te, Fe, Co, Mn, W, Cr, Bi and Zr, suffixes a, b, c, d, e and f each are an atomic ratio of the respective elements,

When b = 12,

a = 0.1 - 3.0, preferably 0.5-1.5

c = 0 - 6.0, preferably 0.1-2.5

d = 0.05 - 5.0, preferably 0.1-2.0

e = 0.01 - 5.0, preferably 0.05-2.0

and f is a numerical value determined according to a valence and atomic ratio of the respective elements.

As catalyst materials various ones may be used.

As phosphorus compounds are cited, for instance, orthophosphoric acid, disodium hydrogenphosphate, monoammonium phosphate, diammonium phosphate and the like. As molybdenum compounds are cited, for instance, molybdenum trioxide, molybdic acid, sodium molybdate, ammonium paramolybdate, phosphomolybdic acid and the like. As vanadium compounds are cited, for instance, vanadium pentoxide, ammonium metavanadate, sodium metavanadate, vanadyl oxalate, vanadyl sulfate and the like..

As X and Y components are cited hydroxides, nitrates, carbonates, halides, ammonium salts and oxyacids, and the like of their respective elements.

As catalyst preparative methods known catalyst preparative methods based on phosphorus-molybdenum or phosphorus-molybdenum-vanadium are all applicable. For instance, predetermined amounts of molybdenum trioxide and vanadium pentoxide are suspended in water and by addition of orthophosphoric acid the mixuture is heated and refluxed with agitation, and a compound of component Y is added, then an aqueous nitrate solution of an alkali metal is added and then the system is heated and concentrated and the resultant clay-like substance is optionally wetted with water, extruded, dried and then calcined in an air stream at 300 to 450° C thereby a catalyst is obtained.

Further, the catalyst may be used by being supported on a suitable carrier. As the carrier there are cited known ones, such as silicon carbide, silica, alpha-alumina, graphite, titania and the like.

In the reaction for production of methacrylic acid or its lower esters by the oxidative-dehydration of isobutyric acid or its lower esters a mixutre of 1.0 to 20 volume %, preferably 3 to 10 volume %, of isobutyric acid or its lower esters with 1.0 to 40 volume %, preferably 5 to 20 volume %, of molecular oxygen is used as a feed gas. As oxygen sources air is industrially advantageous. As diluents inert gases such as nitrogen, carbon dioxide gas, helium and argon, or steam or the like may be used. Particulary the addition of steam is also advantageous for the purpose of suppressing byproducts. A space velocity of the feed gas should suitably be 100 to 5,000 hr$^{-1}$ (STP) and its reaction temperature 200 to 400° C.

In the next place, the present invention will be explained in further detail by way of Examples and Comparative Examples, but the present invention will no way be limited to these examples. The following are definitions of conversion and selectivity used in the instant specification.

Conversion (%) =

$$\frac{\text{Mole numbers of consumed isobutyric acid or its lower ester}}{\text{Mole numbers of fed isobutyric acid or its lower esters}} \times 100$$

Selectivity (%) =

$$\frac{\text{Mole numbers of formed methacrylic acid or its lower esters}}{\text{MOle numbers of consumed isobutyric acid or its lower esters}} \times 100$$

Example 1

Added to 4 l of heated deionized water were 883 g of ammonium paramolybdate and 53 g of ammonium metavanadate to dissolve with agitation.

Added to this solution were 52.4 g of phosphoric acid (85 weight %), successively 400 ml of nitric acid (1.38 in specific gravity) and further, a solution of 81.2 g of cesium nitrate in 500 ml of deionized water, and the mixture was heated and concentrated with agitation. The resultant slurried substance was dried at 250°C for 15 hours for its pulverization, and then by addition of water as a molding aid it was molded in a columnar form 5 mm across x 5 mm long and it was dried at 200°C for 4 hours and then calcined at 400°C for 3 hours in an air stream thereby catalyst I was prepared.

The elementary composition, excepting oxygen, of this catalyst I was, $Mo_{12} P_{1.09} V_{1.09} Cs_{1.0}$ in an atomic ratio. Packed in a steel reaction tube 25.4 mm in diameter was 100 ml of the catalyst so obtained and by passing a mixed gas having the below-mentinoed average composition the reaction was carried out at condition of reaction temperature of 300°C and space velocity of 2,000 $hr^{-1}$ (STP).

| | |
|---|---|
| Isobutyric acid | 5.0 volume % |
| Steam | 5.0 " |
| Oxygen | 6.0 " |
| Others (inert gases based on nitrogen and carbon dioxide gas) | 84.0 " |

The reaction resuts were shown in Table-1-1.

With the progress of a reaction time a lowering of conversion of isobutyric acid was slowly caused, but selectivity to methacrylic acid showed the maximum value at a time after the lapse of 8 hours, and with the lapse of time it, in turn, tended to go lower.

At a time after the lapse of 92 hours the reaction was stopped to conduct regeneration treatment. That is, the feeding of isobutyric acid was stopped, and the system was held for about 3 hours while passing steam, oxygen and other gases as such. The regeneration treatment temperature was set at 300°C, the same as the reaction temperature. after the regeneration treatment was finished, isobutyric acid was fed to resume the reaction. The analysis conducted after 1 hour showed that conversion of isobutyric acid and selectivity to methacrylic acid were both recovered. With the lapse of 70 hours after the reaction was resumed conversion of isobutyric acid and selectivity to methacrylic acid were both seen to go lower and so a second regeneration treatment was carried out. Treating conditions were set as being quite the same as at the previous time.

The reaction was continued for about 400 hours and in the meantime, regeneration treatment was carried out for every about 70 hours. Treating conditions were each set as being the same as the first conditions. As is seen from Table-1-1, in all instances, obviously the effect with regeneration treatment was

4

observed, and it was confirmed that by this treatment method long-term stabilized continuous running is rendered possible.

Table-1-1

| Reaction time (hr) | Conversion of isobutyric acid (%) | Selectivity to methacrylic acid (%) | |
|---|---|---|---|
| 1 | 99.5 | 70.1 | |
| 3 | 99.2 | 72.8 | |
| 8 | 98.7 | 74.5 | |
| 24 | 98.2 | 74.1 | |
| 46 | 98.0 | 74.0 | |
| 70 | 97.8 | 74.0 | |
| 92 | 97.5 | 73.9 | |
| | | | Regeneration treatment (at 300° C for 3 hours) |
| 93 | 98.8 | 74.3 | |
| 117 | 98.3 | 74.5 | |
| 139 | 97.9 | 74.3 | |
| 160 | 97.6 | 74.1 | |
| | | | Regeneration treatment (-ditto-) |
| 161 | 98.5 | 74.3 | |
| 183 | 98.1 | 74.3 | |
| 206 | 97.8 | 74.1 | |
| 233 | 97.5 | 74.0 | |
| | | | Regeneration treatment (-ditto-) |
| 234 | 98.3 | 74.3 | |
| 258 | 98.0 | 74.2 | |
| 278 | 97.9 | 74.0 | |
| 301 | 97.7 | 73.9 | |
| | | | Regeneration treatment (-ditto-) |
| 302 | 98.3 | 74.3 | |
| 324 | 97.9 | 74.0 | |
| 347 | 97.7 | 73.9 | |
| 368 | 97.4 | 73.8 | |
| | | | Regeneration treatment (-ditto-) |
| 369 | 98.2 | 74.2 | |
| 392 | 97.9 | 74.1 | |

Comparative Example 1

Table-1-2 shows the results in the case of carrying out regeneration treatment with a gas not containing steam in the presence of the same catalyst as used in Example 1. That is, in the regeneration treatment of Example 1, the feeding of not only isobutyric acid but also steam was stopped and regeneration treatment was conducted by passing oxygen and other gases alone.

Likewise as in Example 1, regeneration treatment was carried out for every about 70 hours, but no satisfactory effect was observed, and the phenomenon of slow deactivation could not be suppressed.

Table-1-2

| (no H₂O) | | | |
|---|---|---|---|
| Reaction time (hr) | Conversion of isobutyric acid (%) | Selectivity to methacrylic acid (%) | |
| 1 | 99.5 | 70.2 | |
| 7 | 98.8 | 74.5 | |
| 42 | 98.2 | 74.1 | |
| 91 | 97.3 | 73.9 | |
| | | | Regeneration treatment (at 300°C for 3 hours) |
| 92 | 97.2 | 73.6 | |
| 135 | 96.5 | 71.8 | |
| 156 | 96.1 | 71.2 | |
| | | | Regeneration treatment (at 300°C for 6 hours) |
| 157 | 96.3 | 71.1 | |
| 200 | 95.4 | 70.8 | |
| 225 | 95.1 | 70.8 | |
| | | | Regeneration treatment (at 300°C for 9 hours) |
| 226 | 95.5 | 70.8 | |
| 269 | 94.7 | 70.3 | |
| 295 | 94.4 | 70.2 | |

## Example 2

At quite the same conditions as used in Example 1 except that the steam concentration of the regeneration treatment gas was set at 3 volume % or 20 volume %, regeneration treatment with the same catalyst was conducted.

The results were each shown in Table-2-1 and Table-2-2.

Table-2-1

| (H₂O 3 volume %) | | | |
|---|---|---|---|
| Reaction time (hr) | Conversion of isobutyric acid (%) | Selectivity to methacrylic acid (%) | |
| 1 | 99.5 | 70.2 | |
| 93 | 97.4 | 73.8 | |
| | | | Regeneration treatment (at 300°C for 3 hours) |
| 94 | 98.6 | 74.4 | |
| 158 | 97.3 | 73.7 | |
| | | | Regeneration treatment (at 300°C for 5 hours) |
| 159 | 98.7 | 74.3 | |

Table-2-2

| (H$_2$O 20 volume %) | | | |
|---|---|---|---|
| Reaction time (hr) | Conversion of isobutyric acid (%) | Selectivity to methacrylic acid (%) | |
| 1 | 99.5 | 70.1 | |
| 91 | 97.4 | 73.9 | |
| | | | Regeneration treatment (at 300°C for 30 minutes) |
| 92 | 98.2 | 74.3 | |
| 115 | 97.7 | 74.0 | |
| | | | Regeneration treatment (at 300°C for 1 hour) |
| 116 | 98.9 | 74.4 | |
| 185 | 97.5 | 73.9 | |
| | | | Regeneration treatment (at 300°C for 2 hours) |
| 186 | 98.8 | 74.3 | |

Comparative Example 2

At quite the same conditions as used in Example 1 except that the steam concentration of the regeneration treatment gas was set at 0.5 volume %, regeneration treatment with the same catalyst was conducted.

The results were each shown in Table-2-3.

Table-2-3

| (H$_2$O 0.5 volume %) | | | |
|---|---|---|---|
| Reaction time (hr) | Conversion of isobutyric acid (%) | Selectivity to methacrylic acid (%) | |
| 1 | 99.4 | 69.8 | |
| 94 | 97.2 | 73.5 | |
| | | | Regeneration treatment (at 300°C for 3 hours) |
| 95 | 97.3 | 73.5 | |
| 119 | 96.8 | 72.3 | |
| | | | Regeneration treatment (at 300°C for 6 hours) |
| 120 | 97.1 | 72.6 | |
| 143 | 96.5 | 72.0 | |
| | | | Regeneration treatment (at 300°C for 9 hours) |
| 144 | 97.1 | 72.6 | |

Example 3

At quite the same conditions as used in Example 1 except that the oxygen concentration of the regeneration treatment gas was set at 21 volume %, regeneration treatment with the same catalyst was conducted.

The results were each shown in Table-3-1.

Table-3-1

| (O$_2$ 21 volume %) | | | |
|---|---|---|---|
| Reaction time (hr) | Conversion of isobutyric acid (%) | Selectivity to methacrylic acid (%) | |
| 1 | 99.4 | 70.1 | |
| 92 | 97.4 | 73.7 | |
| | | | Regeneration treatment (at 300°C for 1 hour) |
| 93 | 98.3 | 74.1 | |
| 140 | 97.3 | 73.7 | |
| | | | Regeneration treatment (at 300°C for 2 hours) |
| 141 | 98.6 | 74.2 | |

Comparative Example 3

At quite the same conditions as used in Example 1 except that the steam concentration of the regeneration treatment gas was set at 2 volume %, regeneration treatment with the same catalyst was conducted.

The results were shown in Table-3-2.

Table-3-2

| (O₂ 2 volume %) | | | |
|---|---|---|---|
| Reaction time (hr) | Conversion of isobutyric acid (%) | Selectivity to methacrylic acid (%) | |
| 1 | 99.5 | 70.0 | |
| 90 | 97.7 | 73.9 | |
| | | | Regeneration treatment (at 300°C for 3 hours) |
| 91 | 97.2 | 73.9 | |
| 114 | 97.1 | 72.8 | |
| | | | Regeneration treatment (at 300°C for 6 hours) |
| 115 | 97.3 | 73.0 | |
| 137 | 96.7 | 72.3 | |
| | | | Regeneration treatment (at 300°C for 9 hours) |
| 138 | 97.0 | 72.6 | |

Example 4

At quite the same condition as used in Example 1 except that the regeneration treatment temperature were set respectively at 240°C and 360°C, regeneration treatment with the same catalyst was conducted. The results were shown in Table-4-1 and Table-4-2.

Table-4-1

| Reaction time (hr) | Conversion of isobutyric acid (%) | Selectivity to methacrylic acid (%) | |
|---|---|---|---|
| 1 | 99.5 | 70.0 | |
| 92 | 97.5 | 73.9 | |
| | | | Regeneration treatment (at 240°C for 6 hours) |
| 93 | 97.8 | 74.1 | |
| 117 | 97.3 | 73.7 | |
| | | | Regeneration treatment (at 240°C for 12 hours) |
| 118 | 98.0 | 74.3 | |
| 140 | 97.6 | 73.8 | |
| | | | Regeneration treatment (at 240°C for 18hours) |
| 141 | 98.6 | 74.4 | |

9

Table-4-2

| Reaction time (hr) | Conversion of isobutyric acid (%) | Selectivity to methacrylic acid (%) | |
|---|---|---|---|
| 1 | 99.3 | 70.2 | |
| 92 | 97.2 | 73.7 | |
| | | | Regeneration treatment (at 360°C for 30 minutes) |
| 93 | 98.4 | 74.2 | |
| 139 | 97.5 | 73.8 | |
| | | | Regeneration treatment (at 360°C for 1 hour) |
| 140 | 98.8 | 74.4 | |

Comparative Exaple 4

At quite the same conditions as used in Example 1 except that the regeneration treatment temperature were set respectively at 180°C and 450°C, regeneration treatment with the same catalyst was conducted. The results were shown in Table-4-3 and Table-4-4.

Table-4-3

| Reaction time (hr) | Conversion of isobutyric acid (%) | Selectivity to methacrylic acid (%) | |
|---|---|---|---|
| 1 | 99.5 | 70.1 | |
| 92 | 97.4 | 74.0 | |
| | | | Regeneration treatment (at 180°C for 6 hours) |
| 93 | 97.4 | 74.0 | |
| 116 | 96.8 | 72.5 | |
| | | | Regeneration treatment (at 180°C for 12 hours) |
| 117 | 96.7 | 72.3 | |
| 139 | 96.3 | 71.7 | |
| | | | Regeneration treatment (at 180°C for 18hours) |
| 140 | 96.3 | 71.8 | |

10

Table-4-4

| Reaction time (hr) | Conversion of isobutyric acid (%) | Selectivity to methacrylic acid (%) | |
|---|---|---|---|
| 1 | 99.3 | 70.0 | |
| 94 | 97.2 | 73.6 | |
| | | | Regeneration treatment (at 450°C for 30 minutes) |
| 95 | 98.4 | 73.2 | |
| 118 | 97.5 | 72.8 | |
| | | | Regeneration treatment (at 450°C for 1 hour) |
| 119 | 98.8 | 71.7 | |

## Example 5

Catalyst II was prepared in like manner as in Example 1 except that predetermined amounts of rubidium nitrate and potassium nitrate were used instead of cesium nitrate. The atomic composition, excepting oxygen, of this catalyst II was $Mo_{12} P_{1.09} V_{1.09} Rb_{0.7} K_{0.3}$ in an atomic ratio.

Using 100 ml of this catalyst, the oxidation-dehydrogenation reaction of isobutyric acid was carried out by emplying the same method and reaction conditions as in Example 1, and likewise as in Example 1 regeneration treatment was conducted halfway.

The resuts were shown in Table-5.

Table-5

| Reaction time (hr) | Conversion of isobutyric acid (%) | Selectivity to methacrylic acid (%) | |
|---|---|---|---|
| 1 | 96.5 | 68.5 | |
| 24 | 96.0 | 72.3 | |
| 92 | 94.1 | 71.8 | |
| | | | Regeneration treatment (at 300°C for 3 hours) |
| 93 | 96.3 | 72.2 | |
| 160 | 94.0 | 71.5 | |
| | | | Regeneration treatment (-ditto-) |
| 161 | 96.2 | 72.1 | |
| 231 | 93.9 | 71.6 | |
| | | | Regeneration treatment (-ditto-) |
| 232 | 96.3 | 72.2 | |
| 307 | 93.8 | 71.8 | |

### Example 6

Using the catalyst of Example 1, the oxidation-dehydrogenation reaction of methyl isobutyrate was conducted. Packed in a steel reaction tube 25.4 mm in diameter was 100 ml of the catalyst, and by passing a mixed gas having the below-mentioned average composition the reaction was carried out at conditions of reaction temperature of 300° C and apace velocity of 1,500 hr$^{-1}$.

| Methyl isobutyrate | 4.0 volume % |
|---|---|
| steam | 5.0 " |
| Oxygen | 6.0 " |
| Others (inert gases based on nitrogen and carbon dioxide gas) | 85.0 " |

Eight (8) hours after the reaction was initiated conversion of methyl isobutyrate was 81.5 mole %, selectivity to methyl methacrylate was 40.2 mole %, selectivity to methacrylic acid was 36.6 mole % and overall selectivity was 76.8 mole %.

At a time after the lapse of 90 hours regeneration treatment was conducted at 30° C for 3 hours by following the same procedure as in Example 1, in consequence of which its effect was observed, and the same effect was also observed in regeneration treatment for every about 70 hours after that.

### Example 7

1,500 ml of catalyst 1 prepared in Example 1 was packed in a steel reaction tube 24.4 mm in diameter, and by passing a mixed gas having the below-mentioned average composition of reaction was carried out at condition of reaction temperature of 310° C and space velocity of 2,500 hr$^{-1}$.

| Isobutyric acid | 5.0 volume % |
|---|---|
| steam | 5.0 " |
| Oxygen | 8.0 " |
| Others (inert gases based on nitrogen and carbon dioxide gas) | 82.0 " |

The reaction results were shown in Table-6.

Running was carried out for about 4 months, and activity was recovered by regeneration treatment for every 2 weeks, and it was confirmed that by regeneration treatment of the present invention activity persists for long periods of time.

Table-6

| Reaction time (hr) | Conversion of isobutyric acid (%) | Selectivity to methacrylic acid (%) | |
|---|---|---|---|
| 1 | 99.0 | 74.0 | |
| 10 | 96.8 | 72.3 | |
| 17 | 95.5 | 71.0 | |
| | | | Regeneration treatment (at 310° C for 3 hours) |
| 18 | 97.3 | 72.5 | |
| 25 | 96.5 | 71.7 | |
| 32 | 95.1 | 70.5 | |
| | | | Regeneration treatment (at 310° C for 4 hours) |
| 33 | 97.9 | 73.1 | |
| 39 | 96.7 | 72.1 | |
| 47 | 95.3 | 70.5 | |
| | | | Regeneration treatment (-ditto-) |
| 48 | 97.8 | 73.1 | |
| 62 | 95.2 | 70.7 | |
| | | | Regeneration treatment (-ditto-) |
| 63 | 97.8 | 73.0 | |
| 77 | 95.1 | 70.2 | |
| | | | Regeneration treatment (-ditto-) |
| 78 | 97.7 | 73.0 | |
| 92 | 95.0 | 70.2 | |
| | | | Regeneration treatment (-ditto-) |
| 93 | 97.5 | 72.8 | |
| 107 | 94.9 | 70.1 | |
| | | | Regeneration treatment (-ditto-) |
| 108 | 97.3 | 72.5 | |
| 122 | 94.6 | 69.8 | |
| | | | Regeneration treatment (-ditto-) |
| 123 | 97.2 | 72.5 | |

## Claims

1. A process for regenerating a catalyst based on a heteropoly acid containing phosphorus, molybdenum and vanadium and/or an alkali metal salt thereof and/or their partial substitution type compounds which has been used in the production of methacrylic acid or an ester thereof by the oxidative-dehydrogenation of isobutyric acid or an ester thereof, which comprises treating an activity-lowered catalyst with a gas mixture containing 1 to 30 volume % of steam and 5 to 25 volume % of oxygen at a temperature of from 200 to 400°C.

2. A process according to claim 1 in which the treatment is conducted with a gas mixture containing 3 to 20 volume % of steam.

3. A process according to claim 1 or 2 in which the treatment is conducted at a temperature of from 230 to 360°C.

4. A process according to any one of claims 1 to 3 in which the treatment is conducted at a pressure ranging from 0.1 to 1 MPa (1 to 10 atmospheres).

5. A process according to any one of claims 1 to 4 in which the treatment is conducted with the gas mixture at a space velocity of 300 to 5000 hr$^{-1}$ (STP).

6. A process according to any one of the preceding claims which comprises the additional step of using the resulting regenerated catalyst in the production of methacrylic acid or an ester thereof by the oxidative dehydration of isobutyric acid or an ester thereof.

# DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | EP-A-0 255 640 (RÖHM) <br> * Claim 1 * <br> --- | 1-3,6 | B 01 J 27/28 <br> B 01 J 38/06 <br> B 01 J 38/12 <br> C 07 C 51/377 <br> C 07 C 57/04 |
| Y | EP-A-0 071 137 (BAYER) <br> * Claims 1,3,8 * <br> --- | 1-3,6 | |
| A | WO-A-8 806 917 (RÖHM) <br> --- | | |
| A | FR-A-2 498 476 (ASHLAND OIL) <br> --- | | |
| A | DE-B-1 167 348 (LENIA) <br> ----- | | |

TECHNICAL FIELDS
SEARCHED (Int. Cl.5)

B 01 J
C 07 C

The present search report has been drawn up for all claims

| Place of search | Date of completion of the search | Examiner |
|---|---|---|
| THE HAGUE | 14-05-1990 | THION M.A. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P0401)